# EUROPÄISCHE PATENTSCHRIFT

(11) **EP 0 602 424 B1**
(45) Veröffentlichungstag und Bekanntmachung des Hinweises auf die Patenterteilung: **16.04.1997**
(21) Anmeldenummer: 93118948.4
(22) Anmeldetag: 25.11.1993
(51) Int. Cl.: A61M 16/06

(54) **Formkissen für eine Beatmungsmaske**
Cushion member for breathing mask
Coussinet pour masque respiratoire

(30) Priorität: 08.12.1992 DE 4241272
(43) Veröffentlichungstag der Anmeldung: 22.06.1994
(73) Patentinhaber: Continental Aktiengesellschaft, 30165 Hannover (DE)
(72) Erfinder: Musik, Fred, Dipl.-Ing., D-29596 Stadensen (DE); Reger, Uwe, D-22587 Hamburg (DE); Schwarzkopf, Georg, Dipl.-Ing., D-29451 Dannenberg (DE)

(56) Entgegenhaltungen:
- EP-A- 0 294 200
- GB-A- 209 560
- GB-A- 670 357
- US-A- 4 062 357

## Beschreibung

Die Erfindung betrifft ein Formkissen für eine Beatmungsmaske, bestehend aus einem ringförmigen Formteil aus einem weichen elastomerem Kunststoff mit einer Innenwand und einer Außenwand, deren freie Ränder luftdicht miteinander verbunden sind und einen aufblasbaren Wulst bilden, der sich von einer ersten Knicklinie zwischen dem Rand der Innenwand und dem Wulst nach radial innen und von einer zweiten Knicklinie zwischen dem Rand der Außenwand und dem Wulst nach radial außen erstreckt und so zur Abpolsterung der freien Kante der Beatmungsmaske geeignet ist.

Derartige Formkissen sind z.B. aus der GB-A-670 357 bekannt und sind im allgemeinen über eine eingeformte oder eingesetzte Schlauchhülse mit dem gewünschten Luftvolumen befüllbar und mit einem Stöpsel verschließbar. Die Formkissen haben die Aufgabe, eine Abdichtung zum Gesicht der zu beatmenden Person herzustellen, damit der beispielsweise durch Zusammendrücken einer angeschlossenen elastischen Flasche gebildete Beatmungsdruck nicht ohne Wirkung entweicht. Die aus hartem Kunststoff gebildete Maske muß dabei trotz der für die Abdichtung erforderlichen hohen Andruckkraft durch das Formkissen vom Gesicht der zu beatmenden Person beabstandet gehalten werden. Hierfür ist es erforderlich, daß sich der Wulst von der rohrförmigen Abschlußkante der Maske auch radial nach innen erstreckt.

Die bekannten Formkissen dieser Art werden aus Naturkautschuk im Tauchverfahren hergestellt. Dabei wird eine Negativform wiederholt in ein Naturkautschukbad eingetaucht, bis die gewünschte Wanddicke entstanden ist.

Die so hergestellten Formkissen aus Naturkautschuk sind in ihrer Funktion hinsichtlich der Abdichtung und des Schutzes vor der harten Maskenkante nicht zu beanstanden. Da sie jedoch nach Gebrauch immer wieder bei Temperaturen von etwa 130° C sterilisiert werden müssen, werden sie nach und nach einerseits unansehnlich, andererseits neigen sie zum Verkleben. Ihre Lebensdauer ist daher auf relativ wenige Verwendungen mit anschließenden Dampfsterilisationen begrenzt.

An sich in Frage kommende andere Materialien, die eine bessere Widerstandsfähigkeit gegen wiederholte Dampfsterilisationen aufweisen, lassen sich nicht in einem vergleichbaren Verfahren als Formteil herstellen. Es ist daher versucht worden, den geschlossenen Wulst durch ein offenes Profil zu ersetzen. Es hat sich jedoch gezeigt, daß die gewünschte Polster- und Dichtwirkung dabei nicht erzielt wird.

Im Stand der Technik bestand also das Problem, daß die gewünschte Funktion des Formkissens nur mit einem Formteil aus Naturkautschuk erzielbar war, das jedoch bei wiederholter Dampfsterilisation nur eine geringe Lebensdauer aufwies.

Ausgehend von dieser Problemstellung wird gemäß der vorliegenden Erfindung ein Formkissen der eingangs erwähnten Art vorgeschlagen, das dadurch gekennzeichnet ist, daß es als Formteil durch Spritzgießen hergestellt ist und daß der Rand seiner Innenwand gedehnt und mit dem Rand seiner Außenwand verbunden ist.

Zur Lösung des Problems dient ferner ein Verfahren zur Herstellung eines solchen Formkissens, mit folgenden Verfahrensschritten:
- Spritzgießen eines ringformigen Formteils mit einem Rand der Innenwand, einer sich nach radial außen anschließenden Wulstform und einem Rand der Außenwand, wobei der Innenumfang des Randes der Außenwand deutlich größer ist als der Innenumfang des Randes der Innenwand, so daß ein Zwischenraum zwischen den Rändern besteht,
- Aufweiten des Randes der Innenwand zur Anlage am Rand der Außenwand und
- flächiges Verbinden der Ränder von Innenwand und Außenwand.

Erfindungsgemäß wird das Formkissen aus einem weichen elastomeren Kunststoff durch Spritzgießen hergestellt. Dies ist überraschend, da sich durch Spritzen die benötigte Form des Formkissens nicht herstellen läßt. Ein Formkissen der benötigten Formgebung mit einem sich vom Rand auch radial nach innen erstreckenden Wulst läßt sich nicht entformen. Erfindungsgemäß wird daher der Wulst so gebildet, daß er sich nach dem Spritzgießen zunächst ausschließlich nach außen erstreckt. Die Verbindung des Randes der Innenwand mit dem Rand der Außenwand erfolgt erst nach einer Aufdehnung der Innenwand, wodurch die Wulstform aufgrund der auftretenden Materialspannungen nach radial innen gekippt wird, so daß sich der Wulst dann durch das flächige Verbinden des Randes der Innenwand mit dem Rand der Außenwand von dieser Randverbindung aus auch nach radial innen erstreckt.

Der Effekt des durch die Aufdehnung nach innen kippenden Wulstes wird noch dadurch verstärkt, daß die lichte Weite des Randes zum freien Ende hin abnimmt, der Rand sich also zum freien Ende hin verjüngt.

In einer bevorzugten Ausführungsform wird das Kippen des Wulstes nach innen zusätzlich dadurch verstärkt, daß der Rand des Formteils im Untermaß gegenüber der freien Kante der Beatmungsmaske hergestellt ist. Wird das Formteil dann auf dem Rand der Beatmungsmaske befestigt, muß der Rand des Formteils nochmals gedehnt werden, wodurch der Wulst weiter nach innen kippt und seine Gebrauchsform annimmt.

Das erfindugnsgemäße Formkissen ist vorzugsweise aus Silikonkautschuk hergestellt. Zur Verbindung der Ränder von Außenwand und Innenwand sind diese vorteilhaft vollflächig miteinander verklebt.

Die Erfindung soll im folgenden anhand eines in der Zeichnung dargestellten Anwendungs- und Ausführungsbeispieles näher erläutert werden. Es zeigen:
- Figur 1 -: eine Beatmungsmaske mit einem Formkissen und einer angesetzten Beatmungsflasche
- Figur 2 -: einen Schnitt durch das beim Spritzgießen hergestellte Formteil
- Figur 3 -: eine Draufsicht auf das Formteil gemäß Figur 2 auf die Seite der freien Ränder (verkleinert)
- Figur 4 -: einen Schnitt durch das Formteil mit miteinander verklebten Rändern
- Figur 5 -: eine Draufsicht gemäß Figur 3 auf das Formteil gemäß Figur 4
- Figur 6 -: einen Schnitt durch das an der Beatmungsmaske befestigte Formkissen.

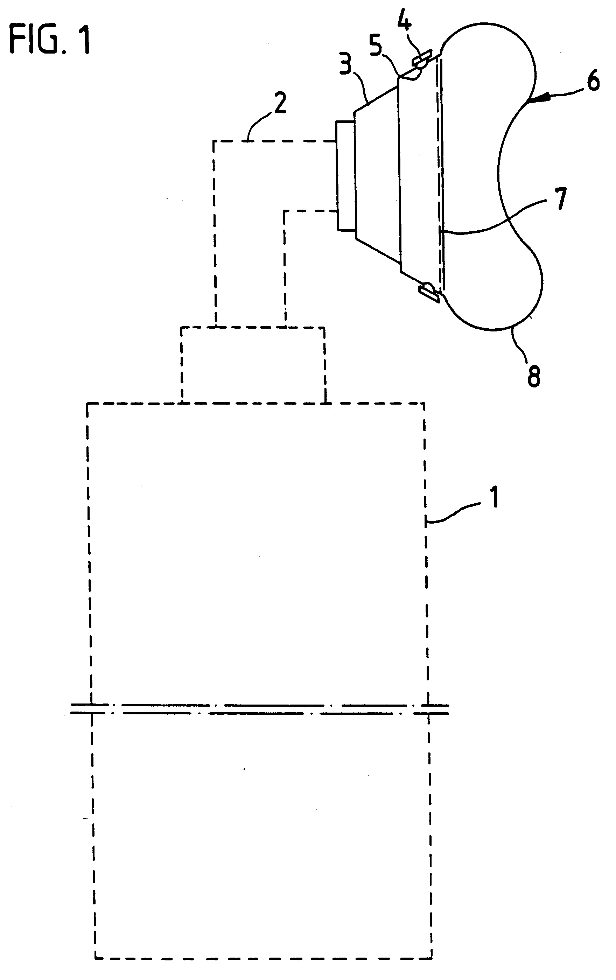

Figur 1 zeigt eine in bekannter Weise mit einem Ventil versehene schematisch dargestellte Beatmungsflasche 1 mit zusammendrückbaren Wänden und einem Anschlußstutzen 2, auf den eine Beatmungsmaske 3 aus hartem Kunststoff aufsetzbar ist. Die Beatmungsmaske 3 weist in an sich bekannter Weise pilzartige Ansätze 4 auf, auf die ein mit entsprechenden Löchern 5 versehenes Formkissen 6 aufknöpfbar ist.

Das Formkissen 6 hat die Aufgabe, die in Figur 1 angedeutete vordere harte Kante 7 der Beatmungsmaske vom Gesicht der zu beatmenden Person fernzuhalten und andererseits einen so großen Andruck am Gesicht der zu beatmenden Person zu ermöglichen, daß eine hinreichende Abdichtung erfolgt. Hierzu weist das Formkissen einen Wulst 8 in einer geeigneten Formgebung auf, die an die anatomischen Gegebenheiten angepaßt ist und die Abdichtung am Gesicht erleichtert.

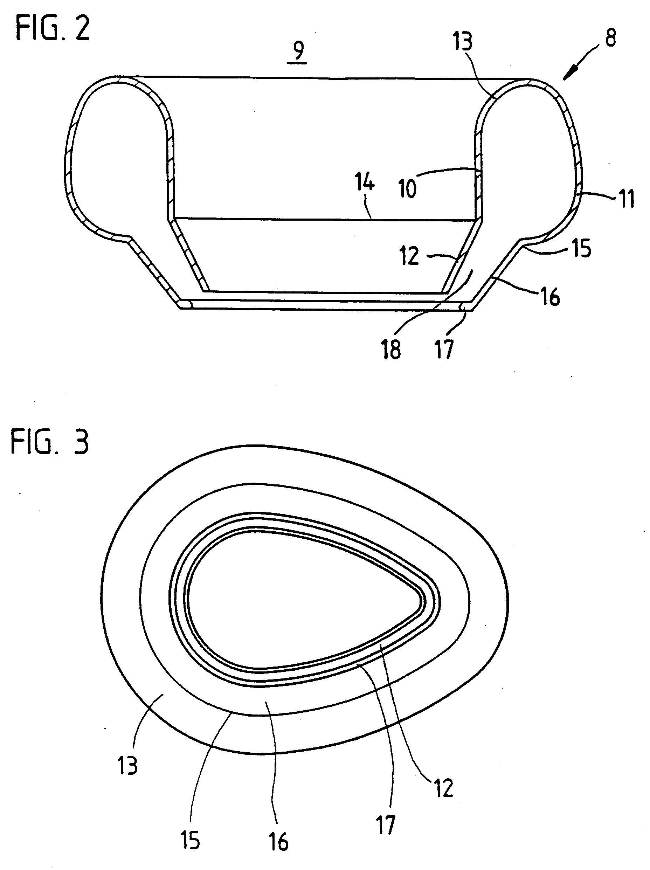

Das erfindungsgemäße Formkissen 6 wird aus einem spritzbaren weichen elastomeren Kunststoff, vorzugsweise Silikonkautschuk, als Formteil 9 hergestellt, das in den Figuren 2 und 3 dargestellt ist.

Das Formteil 9 weist eine Innenwand 10 und eine Außenwand 11 auf.

Die Innenwand 10 besitzt einen freien Rand 12 in Form einer zur Mittelachse des Formteils geneigten Ringfläche, dessen lichte Weite also zum freien Ende hin abnimmt. An den Rand 12 schließt sich eine Wulstform 13 an, die einen Übergang von der Innenwand 10 zur Außenwand 11 bildet. Aus formtechnischen Gründen, nämlich zur Vermeidung von Hinterschneidungen, erstreckt sich die Wulstform 13 von der Knicklinie 14 des Randes 12 zunächst etwa zylindrisch mit gleichbleibendem Durchmesser, um sich dann ausschließlich nach radial außen zur Außenwand 11 zu wölben. Am Ende der etwa ovalen Wölbung geht die Wulstform 13 mit einem Knick 15 in einem Rand 16 der Außenwand 11 über, der ebenfalls eine zur Mittelachse des Formteils geneigte Ringfläche bildet, wobei die Neigung des Randes 16 der Außenwand 11 relativ zur Mittelachse des Formteils 9 größer ist als die Neigung des Randes 12 der Innenwand 10.

Am freien Ende des Randes 16 weist dieser einen schmalen, etwa radial abgewinkelten Randabschluß 17 auf.

Figur 2 verdeutlicht, daß zwischen dem freien Ende des Randes 12 und dem freien Ende des Randes 16 ein deutlicher Zwischenraum besteht.

Ferner ist in Figur 2 erkennbar, daß die Formgebung des Formteils 9 für die Schutzwirkung an der Beatmungsmaske noch nicht geeignet wäre, weil sich die Wulstform 13 ausschließlich nach radial außen erstreckt und beim Anpressen an das Gesicht der zu beatmenden Person nach außen ausweichen würde.

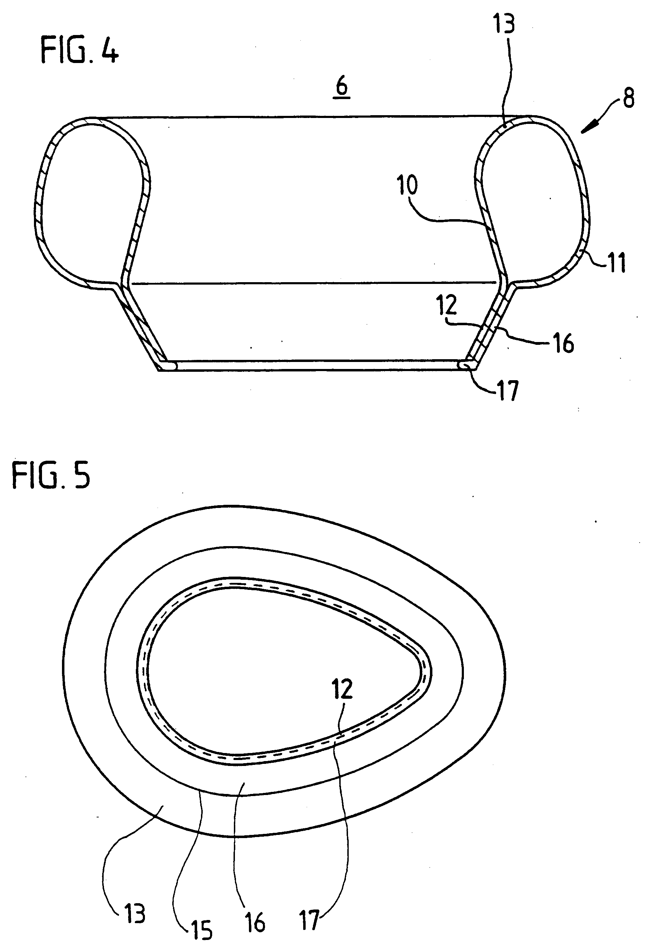

Die Figuren 4 und 5 verdeutlichen die Form des fertiggestellten Formkissens 6. Zur Fertigstellung des Formkissens 6 ist der Rand 12 der Innenwand 10 aufgeweitet und zur Anlage an dem Rand 16 der Außenwand 11 gebracht worden. In dieser Stellung sind die beiden Ränder 12, 16 flächig miteinander verklebt worden, wobei der Randabschluß 17 die Stirnkante des Randes 12 abdeckt. Durch das Aufweiten des Randes 12 der Innenwand 10 und die Verklebung kippt das Wulstteil 13 des Formteils 9 (Figur 2) nach innen und bewirkt, daß der Wulst 8 sich nunmehr von den Knicklinien 14, 15 auch nach radial innen erstreckt. Auf diese Weise gelingt somit die Realisierung einer Form des Wulstes 8, die aus formtechnischen Gründen im Spritzgießverfahren unmittelbar nicht herstellbar wäre.

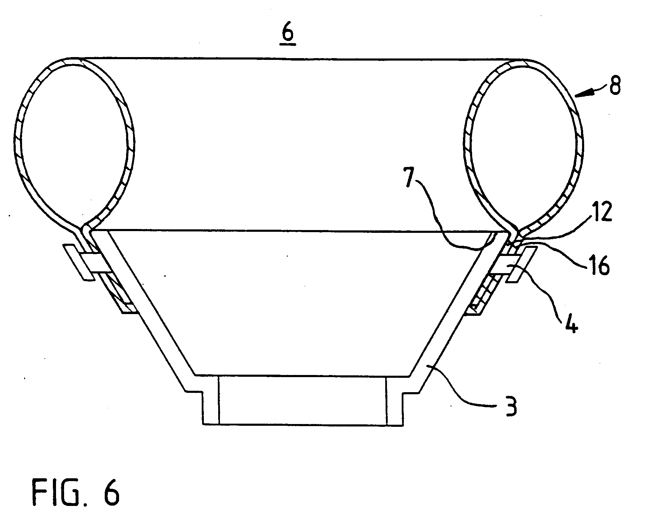

Figur 6 verdeutlicht, daß das in dem dargestellten Beispiel auf die Beatmungsmaske aufgeknöpfte Formkissen 6 einen Wulst 8 aufweist, der sich von der freien Kante der Beatmungsmaske 3 auch nach radial innen erstreckt und insbesondere bei Befüllung mit Luft einen wirksamen Schutz des Gesichtes vor einem direkten Druck durch die Kante 7 der Beatmungsmaske 3 gewährleistet. Figur 6 verdeutlicht gegenüber Figur 4, daß die nochmalige Aufweitung der miteinander verklebten Ränder 12, 16 eine weitere Verformung des Wulstes 8 mit sich bringt, so daß dieser in dem dargestellten Ausführungsbeispiel nach dieser Aufweitung einen relativ zur Kante 7 der Beatmungsmaske 3 annähernd symmetrischen, insbesondere ovalen Querschnitt aufweist.

## Patentansprüche

1. Formkissen (6) für eine Beatmungsmaske (3), bestehend aus einem ringförmigem Formteil (9) aus einem weichen elastomerem Kunststoff mit einer Innenwand (10) und einer Außenwand (11), deren freie Ränder (12, 16) luftdicht miteinander verbunden sind und einen aufblasbaren Wulst (8) bilden, der sich von einer ersten Knicklinie (14) zwischen dem Rand der Innenwand (12) und dem Wulst (8) nach radial innen und von einer zweiten Knicklinie (15) zwischen dem Rand der Außenwand (16) und dem Wulst (8) nach radial außen erstreckt und so zur Abpolsterung der freien Kante (7) der Beatmungsmaske (3) geeignet ist,
**dadurch gekennzeichnet**, daß es als Formteil (9) durch Spritzgießen hergestellt ist und daß der Rand (12) seiner Innenwand (10) gedehnt und mit dem Rand (16) seiner Außenwand (11) verbunden ist.

2. Formkissen nach Anspruch 1, dadurch gekennzeichnet, daß die Ränder (12, 16) als zur Mittelachse des Formteils (9) geneigte Ringflächen ausgebildet sind.

3. Formkissen nach einem der Ansprüche 1 bis 2, dadurch gekennzeichnet, daß der Rand (12, 16) des Formkissens (6) derart dehnbar ist, daß der Wulst (13) bei einer Dehnung des Randes (12, 16) nach innen kippt.

4. Formkissen nach einem der Ansprüche 1 bis 3, dadurch gekennzeichnet, daß die Ränder (12, 16) von Innenwand (10) und Außenwand (11) miteinander verklebt sind.

5. Formkissen nach einem der Ansprüche 1 bis 4, dadurch gekennzeichnet, daß es aus Silikon-Kautschuk besteht.

6. Verfahren zur Herstellung eines Formkissens nach einem der Ansprüche 1 bis 5, gekennzeichnet durch folgende Verfahrensschritte:
- Spritzgießen eines ringförmigen Formteils (9) mit einem Rand (12) der Innenwand (10), einer sich ausschließlich nach radial außen anschließenden Wulstform (13) und einem Rand (16) der Außenwand (11), wobei der Innenumfang des Randes (16) der Außenwand (11) deutlich größer ist als der Innenumfang des Randes (12) der Innenwand (10), so daß ein Zwischenraum (18) zwischen den Rändern (12, 16) besteht,
- Aufweiten des Randes (12) der Innenwand (10) zur Anlage am Rand (16) der Außenwand (11) und
- flächiges Verbinden der Ränder (12, 16) von Innenwand (10) und Außenwand (11).

7. Verfahren nach Anspruch 6, dadurch gekennzeichnet, daß die flächige Verbindung der Ränder (12, 16) durch Verklebung vorgenommen wird.

## Claims

1. Shaped pad (6) for a breathing mask (3), comprising an annular shaped part (9) formed from a soft, elastomeric plastics material and having an internal wall (10) and an external wall (11), the free edges (12, 16) of which walls are interconnected in an airtight manner and form an inflatable bead (8), which extends radially inwardly from a first bend line (14) between the edge (12) of the internal wall and the bead (8) and radially outwardly from a second bend line (15) between the edge (16) of the external wall and the bead (8) and is thus suitable for cushioning the free edge (7) of the breathing mask (3), characterised in that it is produced as a shaped part (9) by injection-moulding, and in that the edge (12) of its internal wall (10) is expanded and connected to the edge (16) of its external wall (11).

2. Shaped pad according to claim 1, characterised in that the edges (12, 16) are configured as annular faces which are inclined towards the central axis of the shaped part (9).

3. Shaped pad according to one of claims 1 to 2, characterised in that the edge (12, 16) of the shaped pad (6) is expansible in such a manner that the bead (8) tilts inwardly when the edge (12, 16) expands.

4. Shaped pad according to one of claims 1 to 3, characterised in that the edges (12, 16) of the internal wall (10) and of the external wall (11) are glued together.

5. Shaped pad according to one of claims 1 to 4, characterised in that it is formed from silicone rubber.

6. Method of producing a shaped pad according to one of claims 1 to 5, characterised by the following method steps:
- injection-moulding an annular shaped part (9) with an edge (12) of the internal wall (10), a bead form (13) which exclusively communicates radially outwardly, and an edge (16) of the external wall (11), the inner circumference of the edge (16) of the external wall (11) being distinctly greater than the inner circumference of the edge (12) of the internal wall (10), so that there is an intermediate space (18) between the edges (12, 16);
- expanding the edge (12) of the internal wall (10) for abutment against the edge (16) of the external wall (11); and
- surface-to-surface bonding between the edges (12, 16) of the internal wall (10) and external wall (11).

7. Method according to claim 6, characterised in that the surface-to-surface bonding of the edges (12, 16) is effected by gluing.

## Revendications

1. Coussin profilé (6) destiné à un masque respiratoire (3), consistant en une pièce profilée annulaire (9) en matière plastique élastomère molle, comprenant une paroi intérieure (10) et une paroi extérieure (11) dont les bords libres (12, 16) sont reliés l'un à l'autre hermétiquement et forment un bourrelet gonflable (8) qui est orienté radialement vers l'intérieur à partir d'une première ligne de coudage (14) située entre le bord (12) de la paroi intérieure et le bourrelet (8) et radialement vers l'extérieur à partir d'une seconde ligne de coudage (15) située entre le bord (16) de la paroi extérieure et le bourrelet (8) et ainsi convient à former un matelas pour l'arête libre (7) du masque respiratoire (3), caractérisé en ce qu'il est réalisé sous la forme d'une pièce profilée (9) par moulage par injection et en ce que le bord (12) de sa paroi intérieure (10) est étiré puis relié au bord (16) de sa paroi extérieure (11).

2. Coussin profilé selon la revendication 1, caractérisé en ce que les bords (12, 16) sont construites sous forme de surfaces annulaires inclinées vers l'axe de symétrie de la pièce profilée (9).

3. Coussin profilé selon l'une des revendications 1 et 2, caractérisé en ce que le bord (12, 16) du coussin profilé (3) est étirable de manière que le bourrelet (13) bascule vers l'intérieur lors de l'étirage du bord (12, 16).

4. Coussin profilé selon l'une des revendications 1 à 3, caractérisé en ce que les bords (12, 16) de la paroi intérieure (10) et de la paroi extérieure (11) sont collés l'un à l'autre.

5. Coussin profilé selon l'une des revendications 1 à 4, caractérisé en ce qu'il est en caoutchouc au silicone.

6. Procédé de réalisation d'un coussin profilé selon l'une des revendications 1 à 5, caractérisé par les étapes suivantes du procédé :
- moulage par injection d'une pièce profilée annulaire (9) comprenant un bord (12) de la paroi intérieure (10), une forme en bourrelet (13) qui se raccorde exclusivement radialement vers l'extérieur et un bord (16) de la paroi extérieure (11), la circonférence interne du bord (16) de la paroi extérieure (11) étant notablement plus grande que la circonférence interne du bord (12) de la paroi intérieure (10), de sorte qu'il subsiste un espace intermédiaire (18) entre les bords (12, 16),
- élargissement du bord (12) de la paroi intérieure (16) pour sa mise en appui contre le bord (16) de la paroi extérieure (11) et
- jonction à plat des bords (12, 16) de la paroi intérieure (10) et de la paroi extérieure (11).

7. Procédé selon la revendication 6, caractérisé en ce que la jonction à plat des bords (12, 16) est réalisée par collage.
